# EUROPÄISCHE PATENTSCHRIFT

(11) **EP 2 602 069 B1**
(45) Veröffentlichungstag und Bekanntmachung des Hinweises auf die Patenterteilung: **13.05.2015**
(21) Anmeldenummer: 12195470.5
(22) Anmeldetag: 04.12.2012
(51) Int. Cl.: B25J 19/06, B25J 13/08

(54) **Kollisionsschutz**
Anti-collision device
Protection contre la collision

(30) Priorität: 09.12.2011 DE 202011052253 U
(43) Veröffentlichungstag der Anmeldung: 12.06.2013
(73) Patentinhaber: Mayser GmbH & Co. KG, 88161 Lindenberg (DE)
(72) Erfinder: Mayer Bernd, 88316 Isny (DE); Speckmann Jürgen, 88161 Lindenberg (DE)
(74) Vertreter: Kaufmann, Sigfrid

(56) Entgegenhaltungen:
- EP-A1- 0 395 784
- EP-A1- 0 696 782
- EP-A2- 1 323 503
- DE-A1- 2 331 465

## Beschreibung

Die Erfindung betrifft ein Bauteil mit einer Kollisionserfassung zum Erfassen einer Kollision zwischen dem Bauteil und einem Gegenstand oder einer Person. Das Bauteil kann z.B. ein motorgetrieben bewegbares Teil einer Maschine sein, etwa ein Roboterarm.

Das Erfassen von Kollisionen ist z.B. in der Medizintechnik und beim Einsatz von Industrierobotern von großer Bedeutung. So muss z.B. beim Einsatz von medizinischen Geräten mit motorgetrieben bewegbaren Bauteilen sichergestellt werden, dass die Bewegung eines solchen Bauteils bei Kontakt bzw. Kollision mit einer Person (z.B. einem mittels des medizinischen Geräts zu untersuchenden Patienten) automatisch angehalten wird. Als ein anderes Beispiel muss beim Einsatz von Industrierobotern mit bewegbaren Bauteilen (z.B. einem Roboterarm) sichergestellt werden, dass die Bewegung eines solchen Bauteils bei einem planmäßigen oder auch außerplanmäßigen Kontakt mit einem Gegenstand (z.B. beim Fassen eines Werkstücks mittels eines Robotergreifarms) oder einer Person (z.B. Wartungspersonal) rechtzeitig angehalten wird.

Zum Erfassen einer Kollision eines flächigen Bauteils können drucksensitive Schaltmatten eingesetzt werden. Die DE 10 2010 010 873 A1 beschreibt die Verwendung einer elektrischen Schaltmatte als drucksensitive Schicht in einer Vorrichtung zur Erkennung einer Kollision, wobei die Schaltmatte z.B. an der Außenseite eines Verkleidungsteils eines medizinischen Geräts angeordnet ist.

Die EP 0 395 784 A1 beschreibt eine elektrische Kontaktmatte mit zwei elektrisch leitenden Platten, die mittels elastisch kompressibler Distanzhalter in einem Abstand zueinander angeordnet sind, wobei zwischen den Platten Kontaktgeber aus einem elektrisch leitenden Material vorgesehen sind, mittels derer bei Druckbelastung der Kontaktmatte ein elektrischer Kontakt zwischen den beiden Platten erfolgt.

Die DE 23 31 465 A1 beschreibt eine Kontaktmatte mit zwei Platten, wobei jede der Platten auf ihrer Innenseite elektrische Kontaktstreifen aufweist, wobei die Kontaktstreifen mittels Rippen aus elektrisch nicht leitendem Material in einem Abstand zu der jeweiligen Platte an derselben gehalten sind.

Die Verwendung einer flächigen Schaltmatte kann jedoch z.B. beim Abdecken von Kanten und unregelmäßigen Flächen eines mit einer kollisionsempfindlichen Außenhülle zu versehenden Bauteils problematisch sein, da an solchen Stellen beim Verlegen der Schaltmatte Überlappungen und Knicke in der Schaltmatte resultieren können, welche das Ergebnis einer Kollisionserfassung verfälschen können und/oder zu Beschädigungen der Schaltmatte führen können. Des Weiteren sind Schaltmatten aufgrund ihrer Steifigkeit oftmals nicht zum formangepassten Abdecken geringer Krümmungsradien geeignet.

Durch die Erfindung wird ein Bauteil mit einer Kollisionserfassung bereitgestellt, das auf einfache Art und Weise herstellbar ist, wobei zudem bei einer beliebigen Form des Bauteils eine zuverlässige Kollisionserkennung gewährleistbar ist.

Gemäß der Erfindung wird ein Bauteil mit einer Kollisionserfassung zum Erfassen einer Kollision des Bauteils bereitgestellt. Das Bauteil weist einen Grundkörper bzw. Bauteil-Grundkörper auf, auf welchen die Kollisionserfassung bzw. Kollisionserfassungsvorrichtung aufgebracht ist. Die Kollisionserfassung weist ein oder mehrere strangförmige drucksensitive Elemente (im Folgenden auch als "Druckdetektoren" bezeichnet) auf, die zum Erzeugen eines Signals bei Druckausübung darauf ausgebildet sind, und die derart an einer Fläche (im Folgenden auch als "Kollisionsschutzfläche" bezeichnet) des Grundkörpers, an der ein Kollisionsschutz vorgesehen ist, angeordnet sind, dass von ihnen die Kollisionsschutzfläche flächig überspannt ist. Zum Beispiel kann vorgesehen sein, dass von den Druckdetektoren eine Fläche des Grundkörpers (z.B. die gesamte Oberfläche des Grundkörpers) einschließlich Rundungen, Schrägen, Ecken und/oder Kanten derselben flächig überspannt ist. Des Weiteren weist die Kollisionserfassung eine Außenhülle auf, die derart ausgebildet und angeordnet ist, dass von ihr die Kollisionsschutzfläche und die drucksensitiven Elemente überdeckt (und z.B. umhüllt) sind. Im Folgenden wird der Einfachheit halber meist im Plural von "Druckdetektoren" gesprochen, womit jedoch die jeweils vorliegenden Detektoren bezeichnet sind und somit auch der Fall lediglich eines einzigen Druckdetektors umfasst sein soll, soweit sich nicht aus dem Kontext etwas anderes ergibt.

Indem die mit einer Kollisionserkennung zu versehende Kollisionsschutzfläche des Grundkörpers mittels strangförmiger bzw. linienförmiger Druckdetektoren überspannt ist, kann bei beliebiger Form der Kollisionsschutzfläche eine formangepasste, flächige Kollisionserfassungsvorrichtung ohne übermäßige Verformung der drucksensitiven Elemente realisiert werden, sodass ein zuverlässiges Erfassen von Kollisionen gewährleistbar ist. So kann z.B. mittels der strangförmigen Druckdetektoren eine Kante des Grundkörpers unter Vermeidung von Überlappungen und/oder scharfkantigen Einknickungen abgedeckt werden, indem ein Druckdetektor entlang der Kante verlaufend angeordnet wird.

Bei einer Kollision des Bauteils, z.B. mit einer Person oder einem Gegenstand als Kollisionspartner, tritt die Außenhülle mit dem jeweiligen Kollisionspartner in Wechselwirkung und überträgt den Kollisionsdruck bzw. die Kollisionskraft (direkt) auf die Druckdetektoren.

Das Bauteil weist ferner eine Schicht (im Folgenden auch als "Abstandsschicht" bzw. "Hinterfütterungsschicht" bezeichnet) aus einem elastisch komprimierbaren Material auf, die auf der Kollisionsschutzfläche des Grundkörpers angeordnet ist; wobei jedes der strangförmigen drucksensitiven Elemente in einer nutförmigen Ausnehmung (im Folgenden auch als "Aufnahmenuten" bezeichnet) der Abstandsschicht aufgenommen ist, und wobei die Außenhülle der Außenkontur der Abstandsschicht formangepasst und (z.B. dieselbe kontaktierend) auf der Abstandsschicht angeordnet ist. Die Abstandsschicht kann eine zusammenhängende oder auch eine aus mehreren voneinander beabstandeten Abschnitten bzw. Teilen bestehende, unzusammenhängende Schicht sein, wobei die Aufnahmenuten durch in der Abstandsschicht ausgebildete nutförmige Aussparungen bzw. durch zwischen zwei voneinander beabstandeten Schichtabschnitten ausgebildete nutförmige Freiräume gebildet sind.

Das Bauteil kann z.B. ein Maschinenteil sein, etwa ein motorgetrieben bewegbares Maschinenteil. Der Grundkörper kann z.B. ein Gehäuseformteil sein und kann z.B. aus einem (im Wesentlichen) starren, unelastischen und/oder nicht komprimierbaren (bzw. inkompressiblen) Material bestehen; wobei das Material des Grundkörpers z.B. einen (wesentlich) größeren Kompressionsmodul aufweisen kann als die Materialien der Abstandsschicht und/oder der Außenhülle. Der Grundkörper kann z.B. aus einem Kunststoff oder einem Metall bestehen. Die Abstandsschicht kann z.B. aus einem Schaummaterial (z.B. einem Blockschaum) oder einem gummielastischen Material bestehen.

Demgemäß sind die strangförmigen Druckdetektoren in den - in bzw. mittels der Abstandsschicht ausgebildeten -Aufnahmenuten aufgenommen, wobei mittels der Abstandsschicht sichergestellt werden kann, dass bei Nichtvorliegen einer Kollision (d.h. bei unkomprimierter Abstandsschicht) von der Außenhülle kein Druck auf die Druckdetektoren ausgeübt wird. Zum Beispiel kann vorgesehen sein, die Aufnahmenuten derart (mit einer entsprechenden Tiefe) auszubilden, dass bei unkomprimierter Abstandsschicht die Druckdetektoren und die Außenhülle voneinander beabstandet sind; wobei die Tiefe der Aufnahmenuten z.B. größer als die Querabmessung (in Richtung der Kollisionsschutzflächen-Normale) der strangförmigen Druckdetektoren gewählt sein kann. Die Tiefe der Aufnahmenuten kann z.B. der Schichtdicke der Abstandsschicht entsprechen (in diesem Fall ist die Bodenseite der nutförmigen Ausnehmungen von der Kollisionsschutzfläche des Grundkörpers gebildet) oder auch geringer als dieselbe sein (in diesem Fall stehen die Druckdetektoren nicht in direktem Kontakt mit der Kollisionsschutzfläche).

Die strang- bzw. linienförmigen Druckdetektoren können z.B. mittels Klebens an der Kollisionsschutzfläche des Grundkörpers bzw. am Boden der jeweiligen Aufnahmenut befestigt sein. Der Begriff "strangförmig" kennzeichnet hier Formen, deren Längsabmessung wesentlich größer ist als deren Querabmessung, wobei die Querschnittsfläche eine beliebige Form aufweisen kann.

Bei einer Kollision des Bauteils, z.B. mit einer Person oder einem Gegenstand als Kollisionspartner, tritt die Außenhülle mit dem jeweiligen Kollisionspartner in Wechselwirkung und überträgt den Kollisionsdruck bzw. die Kollisionskraft zunächst auf die Abstandsschicht und schließlich auch auf die Druckdetektoren. Die Außenhülle bewirkt dabei eine Vergleichmäßigung bzw. flächige Verteilung des Kollisionsdrucks. Während der Kollision bewegt sich die Außenhülle unter Kompression der Abstandsschicht auf den Grundkörper (bzw. die in den Aufnahmenuten aufgenommenen Druckdetektoren) zu. Dabei wird zunächst die elastisch komprimierbare Abstandsschicht komprimiert, wobei mittels des Materials und der Dicke der Abstandsschicht die zum Auslösen der Druckdetektoren erforderliche Druckkraft eingestellt werden kann (zum Beispiel ist die zum Auslösen der Druckdetektoren erforderliche Kraft umso größer, je geringer die Elastizität und je größer die Dicke der Abstandsschicht sind). Mit zunehmendem Kompressionsgrad der Abstandsschicht kontaktiert die Außenhülle schließlich die strangförmigen drucksensitiven Elemente, woraufhin von denselben ein Signal erzeugt wird. Dieses Signal kann erfasst und z.B. mittels einer dafür vorgesehenen Auswerteeinheit ausgewertet werden, wobei mittels der Auswerteeinheit bei Erfassen bzw. Erkennen einer Kollision z.B. ein Warnsignal ausgegeben werden kann.

Das drucksensitive Element bzw. die drucksensitiven Elemente sind derart angeordnet, dass von ihnen die Kollisionsschutzfläche flächig überspannt ist, d.h. die Außenkontur des von den drucksensitiven Elementen abgedeckten Flächenbereichs im Wesentlichen der Umrandung der Kollisionsschutzfläche entspricht. Zum Beispiel kann vorgesehen sein, die Druckdetektoren mäanderförmig und/oder rasterartig über die Kollisionsschutzfläche verlaufend anzuordnen. Es kann auch vorgesehen sein, die Kollisionsschutzfläche mittels mehrerer separater, parallel oder konzentrisch (z.B. kreis- oder ellipsenförmig) zueinander angeordneter strangförmiger Druckdetektoren flächig abzudecken.

Gemäß einer Ausführungsform sind entlang der drucksensitiven Elemente an der dem Grundkörper zugewandten Innenseite der Außenhülle hervorstehende Drucknasen bzw. Druckübertragungsnasen (d.h. vorstehende Ausbuchtungen bzw. Nasen) in einem Abstand zueinander ausgebildet. Die Drucknasen sind somit den Druckdetektoren (bzw. den entsprechenden Aufnahmenuten) gegenüberliegend angeordnet. Mittels der von der Außenhülle in Richtung zu den Druckdetektoren hervorstehenden Drucknasen kann im Falle einer Kollision eine punktuelle Betätigung der strangförmigen Druckdetektoren ermöglicht werden, wobei aufgrund der Verkleinerung der kraftübertragenden Fläche lokal ein großer Druck auf die Druckdetektoren wirkt, wodurch eine Verbesserung des Ansprechverhaltens der Druckdetektoren ermöglicht werden kann.

Gemäß einer weiteren Ausführungsform sind die Drucknasen als längliche Drucknasen bzw. Druckstege ausgebildet, die mit ihrer Längsrichtung quer zur jeweiligen Erstreckungsrichtung des entsprechenden drucksensitiven Elements ausgerichtet sind. Dadurch kann von den Druckstegen entlang der Querrichtung der Druckdetektoren ein großer Wirkungsbereich abgedeckt und zugleich die kraftübertragende Fläche klein gehalten werden. Es kann z.B. vorgesehen sein, die Druckstege mit einer Länge auszubilden, die mindestens so groß ist wie die Querabmessung (parallel zur Kollisionsschutzfläche) der strangförmigen Druckdetektoren, wobei die Länge der Druckstege z.B. bei Ausbildung des Bauteils mit einer Abstandsschicht der Breite der Aufnahmenuten der Abstandsschicht entsprechen kann. So kann auch bei einem seitlichen Verrutschen eines strangförmigen Druckdetektors innerhalb einer Aufnahmenut stets sichergestellt sein, dass im Falle einer Kollision der jeweilige Druckdetektor von einem zugehörigen Drucksteg kontaktiert wird und zuverlässig auslöst.

Gemäß einer weiteren Ausführungsform besteht die Außenhülle aus einem elastisch verformbaren Material, z.B. aus einem (im Wesentlichen) inkompressiblen elastisch verformbaren Material. Zum Beispiel kann vorgesehen sein, bei einer Ausführung des Bauteils ohne eine Abstandsschicht die Außenhülle aus einem elastisch verformbaren Material auszubilden, wobei die Außenhülle bei einer Kollision unter elastischer Verformung direkt auf die Druckdetektoren gepresst wird und nach Entfernen des Kollisionsdrucks aufgrund ihrer Elastizität wieder ihre ursprüngliche Form annimmt und dadurch die Druckdetektoren entlastet.

Gemäß einer weiteren Ausführungsform besteht die Außenhülle aus einem elastisch komprimierbaren Material, z.B. aus einem Schaummaterial oder einem gummielastischen Material. Gemäß dieser Ausführungsform kann die Außenhülle bei einer Kollision als dämpfendes, kraftaufnehmendes Element zum Schutz der Druckdetektoren und/oder des Kollisionspartners vor Beschädigung wirken.

Gemäß einer weiteren Ausführungsform weist die Außenhülle eine geringere Elastizität auf als die Abstandsschicht, verformt sich also bei gleicher Druckeinwirkung weniger als die Abstandsschicht. Zum Beispiel kann das Material der Außenhülle einen größeren Kompressionsmodul aufweisen als das Material der Abstandsschicht.

Gemäß dieser Ausführungsform kann z.B. bei Ausbildung der Außenhülle aus einem elastisch komprimierbaren Material die zeitliche Verzögerung bis zum Auslösen der Druckdetektoren gering gehalten werden, indem bei einer Kollision die weniger elastische Außenhülle zunächst im Wesentlichen unkomprimiert bleibt und lediglich die elastischere Abstandsschicht komprimiert wird, und erst nach der vollständigen Komprimierung der Abstandsschicht bei Kontakt der Außenhülle mit den Druckdetektoren (und somit nach Auslösen der Druckdetektoren) eine (stärkere) Komprimierung der Außenhülle erfolgt.

Gemäß einer weiteren Ausführungsform weist der Grundkörper eine Kante auf, wobei ein strangförmiges drucksensitives Element entlang der Kante verlaufend an dem Grundkörper angeordnet ist. Gemäß dieser Ausführungsform kann zuverlässig eine Kollision mit bzw. an einer Kante des Bauteils erfasst werden; wobei Kollisionen mit Kanten aufgrund der geringen kraftübertragenden Fläche besonders großen Schaden anrichten können. Indem der strangförmige Druckdetektor mit seiner Längsrichtung entlang der Kante verlaufend angeordnet ist, kann die Kante unter Vermeidung einer starken Krümmung des Druckdetektors abgedeckt werden, wodurch ein gleichmäßiges Ansprechverhalten des Druckdetektors entlang der gesamten Kante gewährleistet werden kann, wobei dieses Ansprechverhalten zudem im Wesentlichen identisch zu dem Ansprechverhalten von an einer (im Wesentlichen) ebenen Fläche des Grundkörpers angeordneten Druckdetektoren sein kann.

Gemäß einer weiteren Ausführungsform umfasst das von den drucksensitiven Elementen bei Druckausübung erzeugte Signal eine Änderung einer elektrischen und/oder einer optischen Kenngröße.

Die Druckdetektoren können z.B. elektrische Druckdetektoren sein, wobei das bei Druckausübung erzeugte Signal z.B. eine Änderung eines elektrischen Stroms, eines elektrischen Widerstands, einer elektrischen Spannung und/oder Kapazität sein kann. Zum Beispiel kann vorgesehen sein, die Druckdetektoren als elektrische Schaltleisten auszubilden, die nach dem Schließerprinzip funktionieren; wobei bei Druckausübung ein elektrischer Kontakt geschlossen wird und das Schließen des Kontakts z.B. aufgrund der Änderung eines Stromflusses erfassbar ist.

Es kann auch vorgesehen sein, die Druckdetektoren als optische Druckdetektoren auszubilden, wobei das bei Druckausübung erzeugte Signal z.B. eine Änderung des optischen Transmissionsverhaltens sein kann.

Gemäß einer weiteren Ausführungsform weist das Bauteil mehrere strangförmige drucksensitive Elemente auf, die miteinander wirkverbunden sind. Gemäß dieser Ausführungsform ist es nicht erforderlich, jeden der Druckdetektoren (z.B. durch Verbinden jedes der Druckdetektoren mit einer entsprechenden Auswerteeinheit) separat auszuwerten; vielmehr können die Druckdetektoren gesammelt ausgewertet werden. Beispielsweise kann vorgesehen sein, die einzelnen strangförmigen drucksensitiven Elemente in Reihe zu schalten und zu einem Gesamtstrang zu verbinden, wobei dann lediglich eines der drucksensitiven Elemente mit einer dafür vorgesehenen Auswerteeinheit verbunden werden muss.

Gemäß einer weiteren Ausführungsform ist das Bauteil ein motorgetrieben bewegbares Bauteil und weist eine Auswerteeinheit auf, die mit den drucksensitiven Elementen verbunden ist und derart eingerichtet ist, dass von ihr mittels der drucksensitiven Elemente eine Kollision des Bauteils erfassbar ist und bei Erfassen einer Kollision ein Meldesignal erzeugbar ist und z.B. die Bewegung des Bauteils anhaltbar ist. Es kann auch vorgesehen sein, dass das Bauteil ein manuell oder anderweitig bewegbares Bauteil ist.

Die Auswerteeinheit ist eingerichtet, von den Druckdetektoren erzeugte Signale zu empfangen und anhand der empfangenen Signale zu ermitteln, ob eine Kollision stattgefunden hat oder nicht. Zum Beispiel kann vorgesehen sein, dass von der Auswerteeinheit die von den Druckdetektoren kommenden Signale mit einem Signal-Schwellenwert verglichen werden und ein Überschreiten des Schwellenwertes als Vorliegen einer Kollision gewertet wird. Die Auswerteeinheit kann derart eingerichtet sein, dass das bei Erfassen einer Kollision erzeugte Meldesignal als ein Steuerbefehl-Signal an eine Steuereinheit des motorgetrieben bewegbaren Bauteils übermittelt wird, wobei das Steuerbefehl-Signal z.B. ein Befehl zum Anhalten einer Bewegung des Bauteils oder zum Umkehren einer Bewegungsrichtung des Bauteils sein kann.

Gemäß einer weiteren Ausführungsform ist das Bauteil ein Gehäuseteil; wobei z.B. der Grundkörper ein Gehäuseformteil (z.B. aus einem starren, unelastischen Material) sein kann, das die Gestalt des Bauteils bzw. Gehäuses definiert. Zum Beispiel kann vorgesehen sein, ein an einem technischen Gerät bzw. an einer Maschine vorhandenes Originalgehäuseteil ohne Kollisionserfassungsfunktion durch ein Gehäuseteil gemäß der vorliegenden Ausführungsform mit Kollisionserfassungsfunktion zu ersetzen, wobei zudem durch eine entsprechende Wahl des Materials des Grundkörpers eine Gewichtsreduzierung gegenüber dem Originalgehäuseteil erzielbar sein kann (z.B. wenn das Originalgehäuseteil aus einem Metall besteht und der Grundkörper des ersetzenden Gehäuseteils aus einem Kunststoff gefertigt ist). Des Weiteren kann das Gehäuseteil gemäß der vorliegenden Ausführungsform aufgrund der integrierten Kollisionserkennung mit einer geringeren Stabilität (und somit z.B. mit geringeren Wandstärken und/oder einem geringeren Gewicht) ausgebildet werden als das Originalgehäuseteil, da eine Kollision rechtzeitig erkannt werden kann und somit eine potentielle Beschädigung des Bauteils durch eine solche Kollision z.B. durch Anhalten oder Umkehren einer Bewegung des Bauteils verhindert werden kann und nicht mehr (vollständig) durch das Bauteil abgefangen werden muss.

Die Erfindung wird nachfolgend anhand eines Ausführungsbeispiels mit Bezug auf die beiliegenden Zeichnungen erläutert, wobei gleiche oder ähnliche Merkmale mit gleichen Bezugszeichen versehen sind; hierbei zeigen schematisch:
- Figur 1: eine Querschnittsdarstellung eines Abschnitts eines Bauteils gemäß einer Ausführungsform der Erfindung;
- Figur 2: eine Draufsicht einer Kollisionsschutzfläche des Bauteils gemäß Fig. 1;
- Figur 3: eine Draufsicht einer Innenseitenfläche einer Außenhülle des Bauteils gemäß Fig. 1.

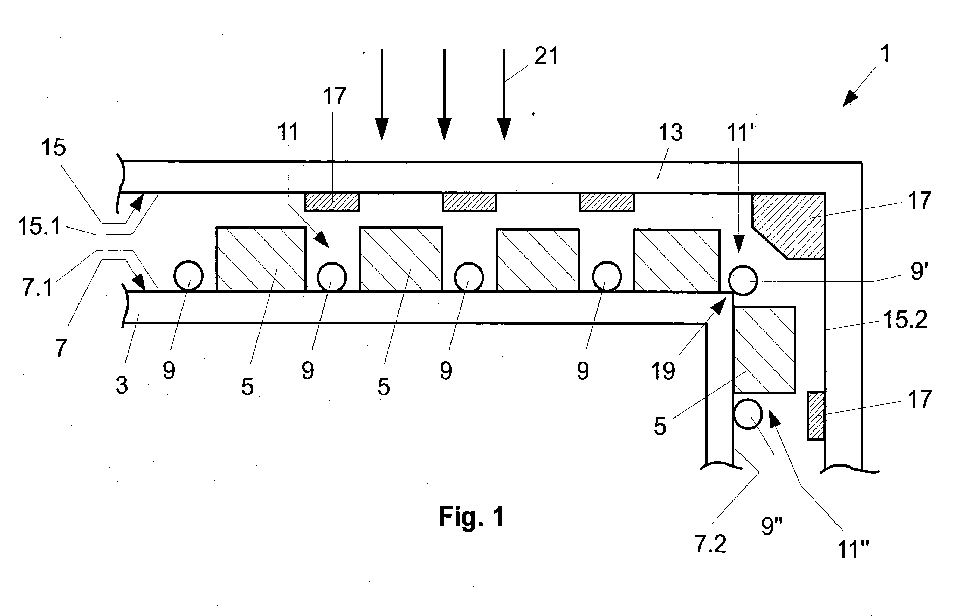

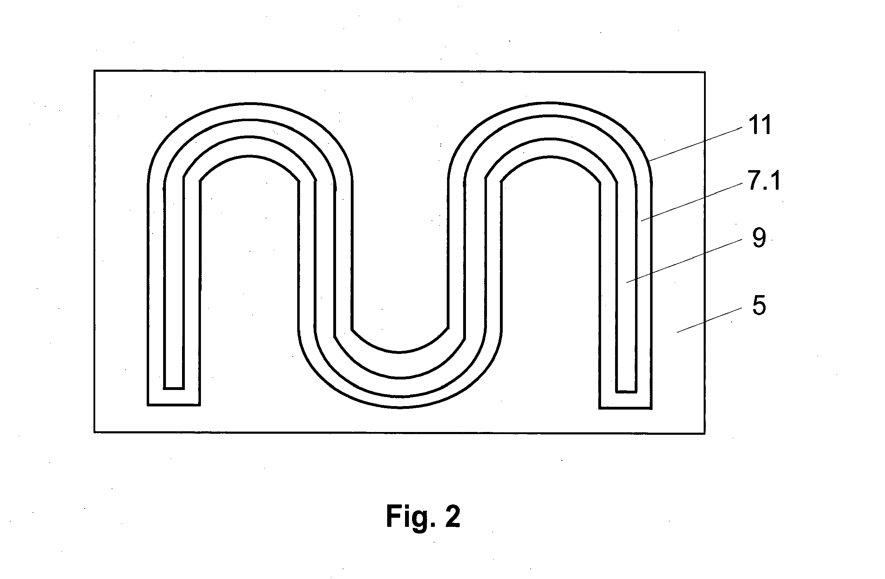

Gemäß den Figuren 1 bis 3 weist ein Bauteil 1 gemäß einer Ausführungsform einen Grundkörper 3 auf. Das Bauteil 1 ist ein einen Gehäuseinnenraum umhausendes Gehäuseteil eines motorgetrieben bewegbaren Maschinenteils (nicht dargestellt) und der Grundkörper 3 besteht aus einem starren, (im Wesentlichen) unkomprimierbaren Material (hier: Kunststoff). Eine Abstandsschicht bzw. Hinterfütterungsschicht 5 aus einem elastisch komprimierbaren Schaummaterial ist auf einer (zu schützenden) Fläche bzw. Kollisionsschutzfläche 7 des Grundkörpers 3 angeordnet. Zudem weist das Bauteil 1 mehrere (hier: drei) strangförmige drucksensitive Elemente bzw. Druckdetektoren 9, 9', 9" auf. Die Druckdetektoren 9, 9', 9" sind derart an der Kollisionsschutzfläche 7 angeordnet, dass von ihnen die Kollisionsschutzfläche 7 flächig überspannt ist. Das Bauteil 1 ist ein Gehäuseteil, wobei der Grundkörper 3 ein die Gestalt des Gehäuseteils 1 definierendes Gehäuseformteil 3 ist, und wobei die Druckdetektoren und die Abstandsschicht an der (dem Gehäuseinnenraum abgewandten) Außenseite des Gehäuseformteils 1 angeordnet sind. Wie in Figur 2 dargestellt, ist als Beispiel der Druckdetektor 9 mäanderförmig derart an dem Horizontalabschnitt 7.1 der Kollisionsschutzfläche 7 angeordnet, dass der Horizontalabschnitt 7.1 von ihm flächig überspannt ist. Jedes der drucksensitiven Elemente 9, 9', 9" ist in einer jeweiligen nutförmigen, in bzw. von der Abstandsschicht 5 ausgebildeten Ausnehmung bzw. Aufnahmenut 11, 11', 11" aufgenommen, wobei die Tiefe der Aufnahmenuten der Schichtdicke der Abstandsschicht 5 entspricht.

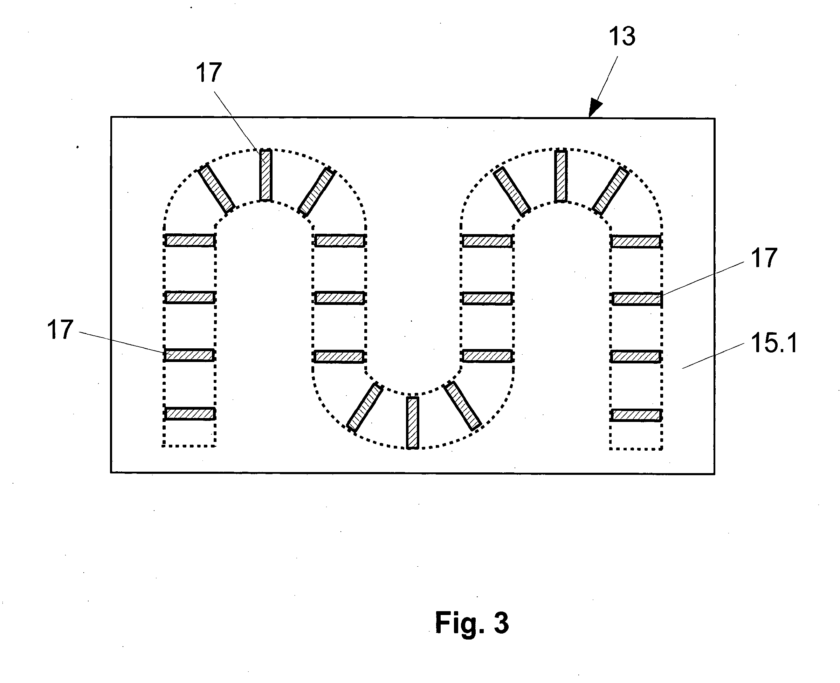

Das Bauteil 1 weist ferner eine Außenhülle 13 auf, die - die Druckdetektoren 9, 9', 9" überdeckend - der Außenkontur der Abstandsschicht 5 formangepasst ist und im Kontakt mit der Abstandsschicht 5 auf derselben angeordnet ist (wobei in Figur 1 der Übersichtlichkeit halber die Außenhülle 13 in einem Abstand von der Abstandsschicht 5 dargestellt ist). An der Innenseite 15 der Außenhülle 13 sind entlang der Druckdetektoren 9, 9', 9" bzw. entlang der zugehörigen Aufnahmenuten 11, 11', 11" Drucknasen in Form von länglichen Druckstegen 17 ausgebildet, die von der Außenhüllen-Innenseite 15 in Richtung zu den Druckdetektoren 9, 9', 9" hervorstehen. Figur 3 veranschaulicht den Horizontalabschnitt 15.1 der Außenhüllen-Innenseite 15, der dem Horizontalabschnitt 7.1 der Kollisionsschutzfläche 7 gegenüberliegt, wobei der Verlauf der gegenüberliegenden Aufnahmenut 11 gestrichelt veranschaulicht ist. Die Druckstege 17 sind jeweils mit ihrer Längsrichtung quer bzw. senkrecht zur jeweiligen Erstreckungsrichtung des gegenüberliegenden Druckdetektors 9 (bzw. der gegenüberliegenden Aufnahmenut 11) ausgerichtet, wobei die Länge der Druckstege 17 der Breite der Aufnahmenut 11 entspricht.

Die Außenhülle 13 ist als elastisch komprimierbares Weichintegralschaumteil ausgebildet, wobei das Schaummaterial der Außenhülle 13 eine geringere Elastizität aufweist als das Schaummaterial der Abstandsschicht 5. Die Außenhülle 13 umgreift bzw. umgibt die Abstandsschicht 5 und die Druckdetektoren 9, 9', 9" und ist an dem Grundkörper 3 befestigt (nicht dargestellt).

Der strangförmige Druckdetektor 9' ist entlang einer Kante 19 des Grundkörpers 3 verlaufend angeordnet. Der Druckdetektor 9" ist an dem Vertikalabschnitt 7.2 der Kollisionsschutzfläche 7 angeordnet und wechselwirkt mit dem Vertikalabschnitt 15.2 der Außenhüllen-Innenseite 15 (bzw. mit den daran angeordneten Druckstegen 17).

Die Druckdetektoren 9, 9', 9" sind als elektrische, nach dem Schließerprinzip funktionierende Detektoren ausgebildet; d.h., bei Ausübung von Druck auf die Druckdetektoren 9, 9', 9" wird ein elektrischer Kontakt zwischen zwei Kontaktelektroden geschlossen, wobei dieser Kontaktschluss eine Änderung der elektrischen Stromstärke des zwischen den zwei Kontaktelektroden fließenden Stromes hervorruft bzw. einen solchen Stromfluss überhaupt erst ermöglicht. Diese Änderung ist als Signal auswertbar.

Das Bauteil 1 bzw. das Gehäuseteil 1 des motorgetrieben bewegbaren Maschinenteils weist eine Auswerteeinheit auf (nicht dargestellt), die mit den Druckdetektoren 9, 9', 9" und mit einer Steuereinheit des bewegbaren Maschinenteils verbunden ist. Bei einer Kollision zwischen dem Horizontalabschnitt der Außenhülle 13 und einem Gegenstand (in Figur veranschaulicht durch die nach unten zeigenden Pfeile 21) wird als Beispiel die Außenhülle 13 in Richtung der Pfeile 21 nach unten gedrückt und bewegt sich unter Kompression der Abstandsschicht 5 auf die Druckdetektoren 9 zu. Mit zunehmendem Kompressionsgrad der Abstandsschicht 5 kontaktieren schließlich die an dem Horizontalabschnitt der Außenhülle 13 angeordneten Druckstege 17 den Druckdetektor 9. Der Druckdetektor 9 löst aus, d.h. erzeugt ein Signal in Form einer Stromstärkeänderung, welches von der Auswerteeinheit erfasst wird. Die Auswerteeinheit ist derart eingerichtet bzw. programmiert, dass von ihr anhand der von den Druckdetektoren 9, 9', 9" erzeugten Signale (in Form der Stromstärkeänderung) ermittelt wird, ob eine Kollision vorliegt oder nicht.

Die Druckdetektoren 9, 9', 9" sind miteinander elektrisch verbunden (nicht dargestellt), sodass die Auswerteeinheit lediglich mit einem dieser Druckdetektoren verbunden sein muss. Bei Erkennen einer Kollision wird von der Auswerteeinheit ein Steuerbefehl-Signal an die Steuereinheit des Maschinenteils übermittelt, mittels dessen ein Anhalten der Bewegung des Maschinenteils bewirkt wird.

### Liste der verwendeten Bezugszeichen

- 1: Bauteil
- 3: Grundkörper
- 5: Abstandsschicht
- 7: Kollisionsschutzfläche
- 7.1: Horizontalabschnitt der Kollisionsschutzfläche
- 7.2: Vertikalabschnitt der Kollisionsschutzfläche
- 9, 9', 9": drucksensitives Element / Druckdetektor
- 11, 11', 11": nutförmige Ausnehmung / Aufnahmenut
- 13: Außenhülle
- 15: Innenseite der Außenhülle
- 15.1: Horizontalabschnitt der Außenhüllen-Innenseite
- 15.2: Vertikalabschnitt der Außenhüllen-Innenseite
- 17: Drucknase / Drucksteg
- 21: eine Kollision veranschaulichende Pfeile

## Patentansprüche

1. Bauteil (1) mit einer Kollisionserfassung zum Erfassen einer Kollision des Bauteils, aufweisend:
- einen Grundkörper (3),
- ein oder mehrere strangförmige drucksensitive Elemente (9, 9', 9"), die zum Erzeugen eines Signals bei Druckausübung darauf ausgebildet sind, und die derart an einer Fläche (7) des Grundkörpers (3) angeordnet sind, dass von ihnen die Fläche des Grundkörpers flächig überspannt ist,
- eine Außenhülle (13), welche die Fläche (7) des Grundkörpers (3) und die drucksensitiven Elemente (9, 9', 9") überdeckt, und
- eine zusammenhängende oder aus mehreren voneinander beabstandeten Abschnitten bestehende Abstandsschicht (5) aus einem elastisch komprimierbaren Material, die auf der Fläche (7) des Grundkörpers (3) angeordnet ist,
- wobei jedes der drucksensitiven Elemente in einer nutförmigen Ausnehmung (11, 11', 11 ") der Abstandsschicht (5) aufgenommen ist, und
- wobei die Außenhülle (13) der Außenkontur der Abstandsschicht (5) formangepasst und auf der Abstandsschicht (5) angeordnet ist.

2. Bauteil nach Anspruch 1, wobei entlang der drucksensitiven Elemente (9, 9', 9") an der dem Grundkörper (3) zugewandten Innenseite (15) der Außenhülle (13) hervorstehende Drucknasen (17) in einem Abstand zueinander ausgebildet sind.

3. Bauteil nach Anspruch 2, wobei die Drucknasen (17) als längliche Druckstege (17) ausgebildet sind, die mit ihrer Längsrichtung quer zur jeweiligen Erstreckungsrichtung des entsprechenden drucksensitiven Elements (9) ausgerichtet sind.

4. Bauteil nach einem der Ansprüche 1 bis 3, wobei die Außenhülle (13) aus einem elastisch verformbaren Material besteht.

5. Bauteil nach Anspruch 4, wobei die Außenhülle (13) aus einem elastisch komprimierbaren Material besteht.

6. Bauteil nach Anspruch 4 oder 5, wobei die Außenhülle (13) eine geringere Elastizität aufweist als die Abstandsschicht (5).

7. Bauteil nach einem der Ansprüche 1 bis 6, wobei der Grundkörper (3) eine Kante (19) aufweist und ein drucksensitives Element (9') entlang der Kante verlaufend an dem Grundkörper angeordnet ist.

8. Bauteil nach einem der Ansprüche 1 bis 7, wobei das Signal eine Änderung einer elektrischen und/oder einer optischen Kenngröße umfasst.

9. Bauteil nach einem der Ansprüche 1 bis 8, wobei das Bauteil mehrere strangförmige drucksensitive Elemente (9, 9', 9") aufweist, und wobei die drucksensitiven Elemente miteinander wirkverbunden sind.

10. Bauteil nach einem der Ansprüche 1 bis 9, wobei das Bauteil (1) ein motorgetrieben bewegbares Bauteil ist, ferner aufweisend eine Auswerteeinheit, die mit den drucksensitiven Elementen (9, 9', 9") verbunden ist und derart eingerichtet ist, dass von ihr mittels der drucksensitiven Elemente eine Kollision des Bauteils erfassbar ist und bei Erfassen einer Kollision ein Meldesignal erzeugbar ist.

11. Bauteil nach einem der Ansprüche 1 bis 10, wobei das Bauteil (1) ein Gehäuseteil (1) ist.

## Claims

1. A component (1) having collision detection for detecting a collision of the component, comprising:
- a base (3),
- one or more strand-shaped, pressure-sensitive elements (9, 9', 9"), which are designed to generate a signal in response to pressure application, and which are disposed on a surface (7) of the base (3) such that said elements extend flatly across the surface of the base,
- an outer cladding (13), which covers the surface (7) of the base (3) and the pressure-sensitive elements (9, 9', 9"), and
- a spacing layer (5), which is made of an elastically compressible material, is either contiguous or comprises a plurality of sections spaced apart from one another, and is disposed on the surface (7) of the base (3),
- wherein each of the pressure-sensitive elements is accommodated in a groove-shaped recess (11, 11', 11") of the spacing layer (5), and
- wherein the outer cladding (13) is designed to match the shape of the outer contour of the spacing layer (5) and is disposed on the spacing layer (5).

2. The component according to claim 1, wherein pressing projections (17), which protrude away from the inner side (15) of the outer cladding (13) facing the base (3), are disposed with spacing between one another along the pressure-sensitive elements (9, 9', 9").

3. The component according to claim 2, wherein the pressing projections (17) are designed as elongated pressing segments (17), which are oriented via the longitudinal direction thereof transversely to the respective extension direction of the corresponding pressure-sensitive element (9).

4. The component according to any one of claims 1 to 3, wherein the outer cladding (13) is made of an elastically deformable material.

5. The component according to claim 4, wherein the outer cladding (13) is made of an elastically compressible material.

6. The component according to claim 4 or 5, wherein the outer cladding (13) has a lower elasticity than the spacing layer (5).

7. The component according to any one of claims 1 to 6, wherein the base (3) has an edge (19), and a pressure-sensitive element (9') is disposed on the base so as to extend along the edge.

8. The component according to any one of claims 1 to 7, wherein the signal comprises a change to an electrical and/or an optical parameter.

9. The component according to any one of claims 1 to 8, wherein the component comprises a plurality of strand-shaped, pressure-sensitive elements (9, 9', 9"), and wherein the pressure-sensitive elements are operatively connected to one another.

10. The component according to any one of claims 1 to 9, wherein the component (1) is a component that can be moved in a motor-driven manner, further comprising an evaluation unit, which is connected to the pressure-sensitive elements (9, 9', 9") and is set up such that said evaluation unit can detect a collision of the component by means of the pressure-sensitive elements and, when a collision is detected, a message signal can be generated.

11. The component according to any one of claims 1 to 10, wherein the component (1) is a housing part (1).

## Revendications

1. Pièce de construction (1) avec un détecteur de collision pour la détection d'une collision de la pièce de construction, comportant :
- un corps de base (3),
- un ou plusieurs éléments en forme de boudins sensibles à la pression (9, 9', 9"), qui, pour la génération d'un signal lors de l'application d'une pression, sont conçus et agencés sur une surface (7) du corps de base (3) de manière à recouvrir toute la surface du corps de base,
- une enveloppe extérieure (13) recouvrant la surface (7) du corps de base (3) et les éléments sensibles à la pression (9, 9', 9"), et
- une couche d'espacement (5) continue ou composée de plusieurs sections espacées les unes des autres, constituée d'un matériau élastiquement compressible, laquelle est agencée sur la surface (7) du corps de base (3),
- dans laquelle chacun des éléments sensibles à la pression est admis dans un évidement en forme de rainure (11, 11', 11 ") de la couche d'espacement (5), et
- dans lequel l'enveloppe extérieure (13) est adaptée de par sa forme au contour extérieur de la couche d'espacement (5) et agencée sur la couche d'espacement (5).

2. Pièce de construction selon la revendication 1, dans laquelle des nez de pression saillants (17), espacés les uns des autres, sont formés sur le côté intérieur (15) de l'enveloppe extérieure (13) tourné vers le corps de base (3), le long des éléments sensibles à la pression (9, 9', 9").

3. Pièce de construction selon la revendication 2, dans laquelle les nez de pression (17) sont conçus comme des tiges de pression allongées (17), lesquelles sont orientées avec leur direction longitudinale transversalement à la direction d'extension respective de l'élément sensible à la pression (9) correspondant.

4. Pièce de construction selon l'une des revendications 1 à 3, dans laquelle l'enveloppe extérieure (13) est constituée d'un matériau élastiquement déformable.

5. Pièce de construction selon la revendication 4, dans laquelle l'enveloppe extérieure (13) est constituée d'un matériau élastiquement compressible.

6. Pièce de construction selon la revendication 4 ou 5, dans laquelle l'enveloppe extérieure (13) présente une élasticité inférieure à celle de la couche d'espacement (5).

7. Pièce de construction selon l'une des revendications 1 à 6, dans laquelle le corps de base (3) comporte une arête (19), et un élément sensible à la pression (9') est agencé sur le corps de base en s'étendant le long de l'arête.

8. Pièce de construction selon l'une des revendications 1 à 7, dans laquelle le signal comprend une modification d'un paramètre électrique et/ou optique.

9. Pièce de construction selon l'une des revendications 1 à 8, dans laquelle la pièce de construction comporte des éléments sensibles à la pression (9, 9', 9") en forme de boudins, et dans laquelle les éléments sensibles à la pression sont fonctionnellement reliés entre eux.

10. Pièce de construction selon l'une des revendications 1 à 9, dans laquelle la pièce de construction (1) est une pièce de construction mise en mouvement par un moteur, comportant en outre une unité d'évaluation reliée aux éléments sensibles à la pression (9, 9', 9") et conçue de manière à pouvoir détecter une collision de la pièce de construction à l'aide des éléments sensibles à la pression, et à ce qu'un signal d'avertissement puisse être généré lors de la détection d'une collision.

11. Pièce de construction selon l'une des revendications 1 à 10, dans laquelle la pièce de construction (1) est une pièce de boîtier (1).
